# EUROPEAN PATENT APPLICATION

(11) **EP 0 523 675 A1**
(43) Date of publication of application: **20.01.1993**
(21) Application number: 92112105.9
(22) Date of filing: 15.07.1992
(51) Int. Cl.: C07K 7/08, C07K 7/10, C07K 15/28, A61K 37/02, A61K 39/395

(54) **Inhibitory agent against HIV superinfection**

(30) Priority: 15.07.1991 JP 174028/91
(71) Applicant: The Calpis Food Industry Co., Ltd., Shibuya-ku Tokyo (JP)
(72) Inventor: Ohki, Kohji, Yokohama-shi, Kanagawa-ken (JP); Ikuta, Kazuyoshi, Sapporo-shi, Hokkaido (JP); Kimura, Takuro, Sapporo-shi, Hokkaido (JP); Ohmura, Kazutaka, Kashiwa-shi, Chiba-ken (JP)
(74) Representative: Herrmann-Trentepohl, Werner, Dipl.-Ing.

(57) **Abstract**

An inhibitory agent against HIV superinfection contains a peptide consisting of an amino acid chain of 66th to 92nd, 68th to 130th or 84th to 102nd amino acids as counted from the N-terminal of human CD4 molecule. The inhibitory agent prevents reinfection with second-round HIV after cells are once infected with HIV. The 84th cystein residue of the peptide having an amino acid chain of 66th to 92nd or 68th to 130th amino acids may be modified with an acetamidomethyl or benzyl group. The inhibitory agent may contain a monoclonal antibody reactable with a peptide of 66th to 92nd or 68th to 130th amino acids or may contain an anti-ideotype antibody produced from an antibody as an antigen reactable with a peptide of 66th to 92nd or 68th to 130th amino acids.

## Description

### BACKGROUND OF THE INVENTION

The present invention relates to an inhibitory agent against HIV (Human Immunodeficiency Virus) superinfection, an antigen for producing an anti-idiotype antibody, and a process for producing an anti-idiotype antibody. More particularly, the present invention relates to an inhibitory agent against HIV superinfection for inhibiting reinfection with second-round HIV after cells are once infected with HIV, an antigen for production of an anti-idiotype antibody utilizable for the inhibitory agent, and a process for producing the anti-idiotype antibody.

Human CD4 proteins are specifically bound with envelope proteins (gp120) of HIV which are known to cause acquired human immunodeficiency syndromes (AIDS) and are known as a receptor capable of leading to infection of T-lymphocytes with HIV (Dalgleish *et al*. Nature 312, 763-767, 1984; Klatzmann *et al*. Nature 312, 767-768, 1984; McDougal *et al*. Science 231,382-385, 1986; Sodroski *et al*. Nature 322, 470-474, 1986; Lasky *et al*. Cell, 50, 975-985, 1987).

It has been made clear that the human CD4 protein is a membrane protein existing on the cell membrane of T-lymphocytes and that the soluble CD4 molecules (soluble molecules formed only from the outer membrame portion of the CD4 molecules) show anti-HIV activities by antagonistically inhibiting the binding of CD4 with gp120. It is also reported that the human CD4 peptide (68-130) possesses anti-HIV activity and is a region specifically bound with the gp120 (Hayashi *et al*. Arch. Virol., 105, 129-135, 1989).

In view of these facts, importance of the infection mechanism via the CD4 molecules in prevention and remedy of AIDS is recognized, and inhibitory agents for initial infection of CD4 positive cells with HIV have been proposed hitherto in various ways. For example, the soluble CD4, anti-CD4 antibody, CD4- peptide, etc. are known, and more particularly, for example, a partial peptide of the CD4 molecule, e.g. a peptide consisting of an amino acid chain of 66th to 92nd amino acids as counted from the N-terminal of the human CD4 molecule and a peptide consisting of an amino acid chain of 68th to 130th amino acids as counted from the N-terminal of human CD4 molecule are proposed. By the way, the amino acid sequence reported by Maddon *et al*. (Cell, 42, 93-104, 1985) was corrected by Littman *et al*. (Cell, 55, 541, 1988) to decrease the number of amino acids by 2 residues from the previous report. We followed herein this corrected sequence.

It is known that since humans have initially been infected with HIV, an asymptomatic persistent infection, i.e. an asymptomatic carrier period (referred to hereinafter as AC period) is continued over an extended period of time, usually, a period as long as about 9.8 years in average until outbreak of AIDS. The mechanism of crisis of AIDS is also discussed to investigate in what route it is outbroken [Production of infectious particles from defective human immunodeficiecy virus type 1 (HIV-1)-producing cell clones by superinfection with infectious HIV-1, M. Yunoki, K. Maotani-Imai, H. Kusuda, M. Motoyama, S. Miyake, H. Imai, Y. S. Shin, S. Kato, K. Sano, C. Morita, M. Nakai, K. Hirai and K. Ikuta, Archives of Virolody 116, 143-158, 1991]. It is reported that in the AC period after initial infection of the CD4 positive cells with HIV, there are cells continuously producing less cytopathic HIV isolate and that these cells are again infected with HIV (this phenomenon being referred to hereinafter as superinfection) to become infective HIV-producing cells which are capable of producing strong cytopathic HIV isolate whereby the CD4 positive cells are rapidly destroyed to permit crisis of AIDS. More precisely, when MT-4 cells which are CD4 positive cell strains are infected with wild type HIV (e.g. HTLV-IIIB, LAV-1, etc.), they exhibit a strong cytopathic activity whereby a number of the cells are dead but a part of the cells remains alive and becomes HIV-persistently infective cells (MT-4/HIV) which show the almost same replication rate as non-infective cells. The majority of HIV produced by the cells remaining alive is found to change into the HIV phenotype so that a number of clones (85% of the whole) are separated by cloning which produce incomplete HIV poor in or devoid of infectivity and cytopathic activity. These phenomena apparently reflect clinical symptoms during the AC period and is considered to be a model of the HIV-existing modes during the AC period. A great number of the MT-4/HIV cells thus formed which produce the incomplete HIV are converted by superinfection with HIV to persistently infective cells without suffering from any cytopathic damage which are capable of producing HIV (wild-type HIV) possessing strong infectivity and cytopathic activity. As the HIV-inhibiting agents proposed hitherto contemplate prevention or remedy of initial infection with HIV, it is necessary to administer the agents at the time of any action where the possibility of infection is regarded higher, for example, at the time of sexual intercourse with patients or infecters, remedy of a number of infecters or patients, conjoint use of injectors, etc. Further, the dose of the agent must be increased since a number of T-cells are infected in a short period of time at the initial infection. Furthermore, possiblity of any side-effect of the agent becomes higher as a large amount of the agent must be administered at a time. Accordingly, the method known hitherto for preventing the initial infection of the CD4 positive cells with HIV is quite insufficient for preventing crisis of AIDS, and so the prevention of the above mentioned superinfection becomes necessary. Under the above circumstances, there is a great demand for developing an inhibitory agent against such superinfection. To say it in another way, crisis of AIDS can be avoided by preventing the human cells from superinfection with HIV even if the cells are initially infected therewith. Thus, the agents showing such effect are expected as an agent for inhibiting crisis of AIDS.

### SUMMARY OF THE INVENTION

Accordingly, it is an object of the present invention to provide an inhibitory agent against HIV superinfection capable of preventing secondary infection (superinfection) of human cells, particularly human CD4 positive cells with second-round HIV after they have once been infected with HIV.

It is another object of the present invention to provide an antigen for producing an anti-idiotype antibody utilizable for an inhibitory agent against HIV superinfection capable of preventing superinfection of human cells, particularly human CD4 positive cells with second-round HIV after they have once been infected with HIV.

It is still another object of the present invention to provide a process for producing an anti-idiotype antibody utilizable for an inhibitory agent against HIV superinfection capable of preventing super infection of human cells, particularly human CD4 positive cells with second-round HIV after they have once been infected with HIV.

The above and other objects of the present invention will become apparent from the following description.

In accordance with one aspect of the present invention, there is provided an inhibitory agent against HIV superinfection comprising an effective amount of a peptide consisting of an amino acid chain of 66th to 92nd, 68th to 130th, or 84th to 102nd amino acids as counted from the N-terminal of human CD4 molecule or of an amino acid chain of 66th to 92nd or 68th to 130th amino acids wherein a sulfydryl group of the cysteine (Cys) residue of the 84th amino acid is modified with an acetamidomethyl (Acm) or benzyl (Bzl) group, whereby after cells are initially infected with HIV, reinfection with second-round HIV is prevented.

In accordance with another aspect of the present invention, there is provided an inhibitory agent against HIV superinfection comprising an effective amount of a monoclonal antibody capable of reacting with a peptide consisting of an amino acid chain of 66th to 92nd or 68th to 130th amino acids as counted from the N-terminal of human CD4 molecule whereby after cells are initially infected with HIV, reinfection with second-round HIV is prevented.

In accordance with still another aspect of the present invention, there is provided an antigen for the production of an anti-idiotype antibody, which comprises an antibody capable of reacting with a peptide consisting of an amino acid chain of 66th to 92nd or 68th to 130th amino aicds as counted from the N-terminal of human CD4 molecule, the anti-idiotype antibody functioning so that after cells are initially infected with HIV, reinfection with second-round HIV is prevented.

In accordance with further aspect of the present invention, there is provided an inhibitory agent against HIV superinfection which comprises an effective amount of an anti-idiotype antibody prepared from the above mentioned antigen for producing the anti-idiotype antibody whereby after cells are initially infected with HIV, reinfection with second-round HIV is prevented.

In accordance with still further aspect of the present invention, there is provided a process for producing an anti-idiotype antibody which comprises using as an immunogen an antibody reactable with a peptide consisting of an amino acid chain of 66th to 92nd or 68th to 130th amino acids as counted from the N-terminal of human CD4 molecule, the anti-idiotype antibody functioning so that after cells are initially infected with HIV, reinfection with second-round HIV is prevented.

### BRIEF DESCRIPTION OF THE DRAWINGS

Fig. 1 is a graph showing occurrence of superinfection by the presence or absence of the reaction with a p-18 antigen in the test examples of the present invention.

Fig. 2 is a graph showing the result measured according to the fluorescent antibody technique using the V-17 antibody as to whether or not the effective ingredient of each inhibitory agent prevents superinfection in the test examples of the present invention.

Fig. 3 is a graph showing the result measured according to the fluorescent antibody technique using the V-17 antibody as to whether or not the effective ingredient of each inhibitory agent prevents superinfection in the test examples of the present invention.

### DESCRIPTION OF THE PREFERRED EMBODIMENTS

The present invention will now be explained in more detail hereunder.

The inhibitory agent against HIV superinfection of the present invention contains a peptide consisting of a specific amino acid chain, a monoclonal antibody capable of reacting with a peptide consisting of a specific amino acid chain, or an anti-idiotype antibody prepared from an antigen for producing the anti-idiotype antibody and being capable of reacting with a peptide consisting of a specific amino acid chain, and prevents reinfection (superinfection) with second-round HIV after cells, particularly human CD4 positive cells are infected with HIV, thereby exhibiting prevention effect against crisis of AIDS in the AC period. When cells are initially infected with wild-type HIV, strong cytopathic activity is exhibited so that the majority of the cells are killed. In this case, the cells remaining alive produce incomplete HIV which are poor in or devoid of infectivity and cytopathic activity and such cells persistently infected with HIV exhibit the same replication rate as non-infected cells. The human cells infected with HIV are meant by such cells. On reinfection with HIV (superinfection), these cells are converted without suffering from any cytopathic damage to persistently infected cells capable of producing wild-type HIV possessing infectivity and cytopathic activity. More precisely, for example, the existence of L-2 cells, L-3 cells, H-7 cells, etc. is detected as such cells (Yunoki, *et al*., Arch. Viral. 116,143-158, 1981). The L-2 cells can be obtained easily, for example, by infecting MT-4 cells with LAV-1 which is a wild-type HIV (F. Barre Sinoussi, *et al*., Science, 220, 868-871, 1983), diluting the infected cells according to the limiting dilution method with an RPMI-1640 medium containing 20% cow embryonic serum, pouring the medium onto a microplate in such manner that each well contains one cell, and culturing it for 2 to 3 weeks. The L-2 cell is one of the LAV-1 persistently infected clones and is known to be a cell in the form of a doughnut producing defective HIV particles wherein gag precursor protein p53 of HIV remaining unripened is not yet cleaved, P24, P17, P9 and P7 which are ripened gag proteins are not detected, and the reverse transcriptase (RT) activity is negative (Goto, *et al*., Arch.Viral. 111, 87-101, 1990).

The peptide used for the inhibitory agent against HIV superinfection consists of a specific amino acid chain of (1) 66th to 92nd amino acids, (2) 68th to 130th amino acids, (3) 84th to 102nd amino acids, (4) Cys at 84th being modified with Acm (acetamidomethyl) of 66th to 92nd amino acids, (5) Cys at 84th being modified with Bzl (benzyl) of 66th to 92nd amino acids,(6) Cys at 84th being modified with Acm of 68th to 130th amino acids or (7) Cys at 84th being modified with Bzl of 68th to 130th amino acids,and is represented by the following amino acid sequence:

(1) Asn-Phe-Pro-Leu-Ile-Ile-Lys-Asn-Lue-Lys-Ile-Glu-Asp-Ser-
Asp-Thr-Tyr-Ile-Cys-Glu-Val-Glu-Asp-Gln-Lys-Glu-Glu (66th to
92nd)
(2) Pro-Leu-Ile-Ile-Lys-Asp-Leu-Lys-Ile-Glu-Asp-Ser-Asp-Thr-
Tyr-Ile-Cys-Glu-Val-Glu-Asp-Gln-Lys-Glu-Glu-Val-Gln-Leu-Leu-
Val-Phe-Gly-Leu-Thr-Ala-Asn-Ser-Asp-Thr-His-Leu-Leu-Gln-Gly-
Gln-Ser-Leu-Thr-Leu-Thr-Leu-Glu-Ser-Pro-Pro-Gly-Ser-Ser-Pro-
Ser-Val-Gln-Cys (68th to 130th)
(3) Cys-Glu-Val-Glu-Asp-Gln-Lys-Glu-Glu-Val-Gln-Leu-Leu-Val-
Phe-Gly-Leu-Thr-Ala (84th to 102nd)
(4) Asn-Phe-Pro-Leu-Ile-Ile-Lys-Asn-Leu-Lys-Ile-Glu-Asp-Ser-
Asp-Thr-Tyr-Ile-Cys(Acm)-Glu-Val-Glu-Asp-Gln-Lys-Glu-Glu
(5) Asn-Phe-Pro-Leu-Ile-Ile-Lys-Asn-Leu-Lys-Ile-Glu-Asp-Ser-
Asp-Thr-Tyr-Ile-Cys(Bzl)-Glu-Val-Glu-Asp-Gln-Lys-Glu-Glu
(6) Pro-Leu-Ile-Ile-Lys-Asn-Lue-Lys-Ile-Glu-Asp-Ser-Asp-Thr-
Tyr-Ile-Cys(Acm)-Glu-Val-Glu-Asp-Gln-Lys-Gln-Gln-Val-Gln-
Leu-Leu-Val-Phe-Gly-Leu-Thr-Ala-Asn-Ser-Asp-Thr-His-Leu-Leu-
Gln-Gly-Gln-Ser-Leu-Thr-Leu-Thr-Leu-Glu-Ser-Pro-Pro-Gly-Ser-
Ser-Pro-Ser-Val-Gln-Cys
(7) Pro-Leu-Ile-Ile-Lys-Asn-Leu-Lys-Ile-Glu-Asp-Ser-Asp-Thr-
Tyr-Ile-Cys(Acm)-Glu-Val-Glu-Asp-Gln-Lys-Glu-Glu-Val-Gln-
Leu-Leu-Val-Phe-Gly-Leu-Thr-Ala-Asn-Ser-Asp-Thr-His-Leu-Leu-
Gln-Gly-Gln-Ser-Leu-Thr-Leu-Thr-Leu-Glu-Ser-Pro-Pro-Gly-Ser-
Ser-Pro-Ser-Val-Gln-Cys

In addition, the following peptides can also be mentioned as preferable ones:

(8) Asn-Phe-Pro-Leu-Ile-Ile-Lys-Asn-Leu-Lys-Ile-Glu-Asp-Ser-
Asp-Thr-Tyr-Ile-Phe-Glu-Val-Glu-Asp-Gln-Lys-Glu-Glu
(9) Asn-Phe-Pro-Leu-Ile-Ile-Lys-Asn-Leu-Lys-Ile-Glu-Asp-Ser-
Asp-Thr-Tyr-Ile-Ser-Glu-Val-Glu-Asp-Gln-Lys-Glu-Glu.

In this invention, the peptides and the modified peptides represented by the above formulas (1) to (9) can be prepared according to the following processes:

As a first process, the peptides can be obtained according to the solid phase method (Sheppard, R.C. *et al*., J. Chem. Comm., 165-166, 1985) using Fmoc amino acids. Firstly, a Fmoc amino acid pentafluorophenyl(Pfp) ester corresponding to the C-terminal of any of the peptide (1) to (9) is bound in a solvent, e.g.dimethylformamide in the presence of 4-dimethylaminopyridine or the like to a high molecular carrier such as a p-alkoxybenzyl alcohol resin. The Fmoc amino acid Pfp ester to be bound next is then subjected to condensation reaction with the high molecular carrier. In this case, it is preferred that threonine and serine are introduced by the use of their 1-oxo-2-hydroxydihydrobenzotriazine (DHBT) ester. After completion of the coupling reaction of each amino acid, the high molecular carrier to which the peptide has been bound is treated with a mixture of piperidine and dimethylformamide or the like to eliminate the N-terminal Fmoc group and then reacted with a trifluoroacetate (TFA), thioanisole and the like at room temperature in the presence of m-cresol to split off all the protective groups while the peptide aimed at is recovered from the high molecular carrier, which can then be purified. In case a peptide containing an arginine residue is obtained, it is treated with trimethylsilyl bromide (TMSBr)/TFA.

As a second process, the peptides can be obtained according to the solid phase method using a t-Boc amino acid (Merrifield, J. Am. Chem. Soc. 85, 2149, 1963). In this second process, a peptide having a modified side chain of an amino acid as in the case of the modified peptides (4) to (7) can be obtained by introducing a stable modified amino acid into the peptide under conditions for splitting off protective groups.

In addition to the above mentioned processes, these peptides can be obtained by other chemical processes usually carried out wherein a DNA corresponding to the peptide to be prepared is first obtained and inserted into a proper vector and is reproduced in animal cells or microorganisms to obtain a partial peptide aimed at and wherein the resultant peptide is chemically modified to obtain the end peptide.

The monoclonal antibody capable of reacting with a peptide consisting of a specific amino acid chain, which is used for the inhibitory agent of this invention, can be produced by synthesizing the above peptide (1) or (2), immunizing an animal with the peptide as an immunogen, subjecting the lymphocytes of the animal and myeloma cells to cell fusion to form a hybridoma strain, and allowing the strain to produce the antibody. The hybridoma as a clone strain capable of reacting with human CD4 peptide (68-130) was deposited as Hybridoma CP-9 (FERM P-10864 BP-3011) while that as a clone strain capable of reacting with human CD4 peptide (66-92) was deposited as Hybridoma CP-1 (FERM P-10862 BP-3009).

More precisely, the peptide (1) or (2) functioning as an antigen is firstly synthesized and the resultant peptide or a peptide which is obtainable by binding the resultant peptide with a carrier protein such as bovine serum albumin or hemocyanine is immunized with a mammal, for example, mouse or rat. In this case, the mouse is preferably of 5 to 10 week age. The antigen is administered in mixture with an adjuvant such as complete Freund's adjuvant, incomplete Freund's adjuvant or alum adjuvant to the mouse in an amount of 0.01 to 1 mg, in particular 50-200 µg each time. This administration is carried out, for example, by way of hypodermal, intravenous or intraperitoneal injection. The antigen is administered to the mouse for additional immunization several times, more specifically, 3 to 4 times at an interval of 1 to 3 weeks whereby the complete immunization is achieved. Lymphocytes are extracted from the animal thus immunized 2 to 4 days after the final immunization. Such organs as spleen, lymphoid nodule or peripheral blood may be used for the preparation of the immunized lymphocytes, and the lymphocytes are then fused with myeloma cells.

As the myeloma cells, cells devoid of specific enzymes such as, for example, mouse myeloma P3-X63-Ag8-U1(P3-U1), P3-X63-Ag8-653(X63-653), P3-NS1-1-Ag4-1(NS-1), or rat myeloma 210-RCY3-Ag123 are preferred. This cell fusion may be carried out in accordance with the Koeler and Milstein's method (Nature 256, 495-497, 1975).

After completion of the cell fusion, hybridomas are selected by using a selective culture medium, for example, HAT (hypoxathine-aminopterin-thymidine), and those capable of producing an antibody against human CD4 peptide are selected by the enzymatic immunoassay. The hybridoma cells capable of producing the monoclonal antibody can be produced by repeating cloning according to the limitation dilution method 1 to 3 times. The monoclonal antibody aimed at can be obtained by inoculating the abdominal cavity of the animal of the same genus with the hybridoma cells thus produced and collecting the abdominal dropsy after multiplication or by cultivating the cells in a cultivator.

In case a human type monoclonal antibody is required, such antibody may be obtained as well according to an *in vitro* immunization method. More precisely, lymphatic cell fractions are prepared from the peripheral blood according to the specific gravity centrifugal method by the aid of Ficoll-Paque (Farmacia Fine Chemical Co.) or the red blood cell removal method by ammonium chloride, or according to the flow cytometry. The cell fraction thus obtained is cultivated and the above peptide (1) or (2) is added to the culture liquid, for example, at a concentration of 0.001 - 1 mg/ml to cause the macrophage to undergo antigen presentation. The co-existent helper T-cells are activated by the presented antigen to transfer the antigen information to the B-cells to produce the B-cells sensitized by the antigen, i.e. the B-cells capable of producing the antibody against the peptide (1) or (2). The B-cells are then changed into strains by the aid of EB (Epstein-Barr) virus or by cell fusion with myeloma, thereby making it possible to produce the polyclonal antibody persistently. The B-cells thus changed into strains may be separated according to a method wherein cloning is carried out by 2 to 3 times according to the limiting dilution method to obtain the monoclonal antibody.

The direct reactivity of the monoclonal antibody used in the inhibitory agent of this invention with the CD4 protein existing in the membrane of the CD4 positive cells such as MOLT-4 or MT-4, can be confirmed by investigating it according to the western blotting method. For example, about 2 × 10⁷ of the CD4 positive cells are collected and treated by homogenization in a hypotonic phosphate buffer containing a protease inhibitor such as phenylmethyl sulfonyl fluoride (PMSF) to destroy the cells, and the mixture is subjected to a centrifugal treatment at 1,000 rpm for 5 minutes to obtain a supernatant separated from the undestroyed cell fraction precipitated. The supernatant is then subjected to centrifugal treatment at 6,500 rpm for 20 minutes to obtain a crude nuclear fraction containing as a precipitate the cell membrane, and this crude nuclear fraction is separated by electrophoresis using SDS-10% polyacrylamide gel according to Laemmli *et al*. method (Nature, 227, 680, 1970) and then subjected to the western blotting according to Towbin *et al*. method (Proc.Natl. Acad. Sci. USA, 76, 4350-4354, 1979). Using the above monoclonal antibody as the primary antibody, coloration is made with a secondary antibody bound with a peroxidase whereby a specific color band is observed at a region corresponding to the molecular weight range of the CD4 molecule from 55000 to 60000. This color band can also be detected in case of using a commercially available anti-CD4 monoclonal antibody OXT4 (Ortho Diagnostic Systems, Inc.). Accordingly, the monoclonal antibody of the present invention is capable of being bound with cell-derived CD4 protein and recognizes the specific binding site with the HIV which is necessary for HIV superinfection after human dells have been initially infected with HIV, so that the monoclonal antibody can effectively prevent HIV superinfection.

The anti-idiotype antibody produced from the antigen for producing anti-idiotype antibody capable of reacting with the peptide consisting of a specific amino acid chain can be produced from an antibody as an immunogen which is capable of reacting with a peptide consisting of the above amino acid chain (1) or (2). In order to produce the anti-idiotype antibody, for example, the peptide consisting of the above amino acid chain (1) or (2) is synthesized at the first stage and an antigen for producing the anti-idiotype antibody, which is capable of reacting with the peptide, i.e. the above monoclonal antibody or polyclonal antibody is prepared at the second stage. The monoclonal antibody or polyclonal antibody alone may be an antigen for producing the anti-idiotype antibody but may preferably be bound to a carrier protein such as hemocyanine to prepare an antigen for producing the anti-idiotype antibody. The antigen for producing the anti-idiotype antibody may be purified as needed. More particularly, ammonium sulfate fractionation, ion exchange, gel filtration, affinity chromatography and the like methods utilizable for ordinary proteins can be used for the purpose of purification.

At the third stage for producing the anti-idiotype antibody, an animal is immunized with the antigen for producing the anti-idiotype anibody. This immunizing method may be in principle carried out in the same manner as in the immunizing method for the monoclonal antiboy. Namely, the animal of the same genus as in the production of the antibody is immunized with the antigen for producing the anti-idiotype antibody. In case a mouse is used as the animal, for example, each mouse is preferably immunized with the antigen 4 to 5 times each in an amount of 0.1 to 1 mg at an interval of 1 to 2 weeks.

The anti-idiotype antibody may be produced from the polyclonal antibody, but the monoclonal antibody with uniform epitope may preferably be used.

In other methods, the anti-idiotype antibody against each peptide can be prepared, using peripheral blood B-lymphocytes infected with HIV. In an immunological system infected with HIV, the major region of the HIV envelope protein is already recognized as the antigen determinant. On the other hand, the anti-idiotype antibody against each peptide is considered to be a specific form of the antibody against the HIV envelope protein. The anti-idiotype antibody behaves similarly to the anti-HIV envelope protein antibody capable of reacting with the specific binding site on the HIV envelope protein with the CD4 protein. Accordingly, B-lymphocytes are produced by the peripheral blood infected with the HIV and are stimulated and sensitized by using a proper secondary antigen, for example, the above antigen for producing the anti-idiotype antibody whereby only the cells capable of producing the antibody corresponding to the anti-idiotype antibody is stimulated by the antigen and multiplied. These multiplied cells are changed into strains by EB virus or the like and cultivated to obtain the anti-idiotype antibody.

Among the anti-diotype antibodies utilizable for the inhibitory agents of this invention against HIV superinfection, the anti-idiotype antibody produced from the antigen for producing the anti-idiotype antibody which is capable of reacting with a peptide consisting of an amino acid chain of 68th to 130th amino acids and capable of inhibiting HIV superinfection after infection of the human cells with HIV, is deposited as ID-55 (FERM P-11157, BP-3165) while the anti-idiotype antibody produced from the antigen for producing the anti-idiotype antibody which is capable of reacting with a peptide consisting of an amino acid chain of 66th to 92nd amino acids and capable of inhibiting HIV superinfection after infection of the human cells with HIV, is deposited as ID-62 (FERM P-11158, BP-3166).

The inhibitory agents against HIV superinfection of this invention contain a peptide consisting of a specific amino acid chain, a monoclonal antibody capable of reacting with the peptide, or an anti-idiotype antibody produced from an antigen for producing the anti-idiotype antibody capable of reacting with the peptide, and is admistered, for example, as such or may be used as a targeting probe for infected cell-killers by being bound to a cytotoxicant.

The inhibitory agent of this invention for inhibiting HIV superinfection is administered to HIV-infected patients in an amount effective to inhibit HIV superinfection during the AC period. The amount of HIV on superinfection is as low as 1/10 - 1/100 in concentration as compared with the amount of HIV in case of the initial infection of human cells with HIV. Accordingly, the amount of administering the inhibitory agent is as low as 1/10 - 1/100 in the amount as compared with the HIV-inhibitory agents proposed hitherto. More precisely, the amount in case of the effective ingredient being the peptide is preferably 50 - 1500 mg/day in terms of the amount of the peptide. In case the effective ingredient is the monoclonal antibody or anti-idiotype antibody, the amount is preferably 50-2000 mg/day in terms of the amount of the antibody. In case of inhibiting superinfection, the infection period is longer than the initial infection period. It is therefore possible to effect continuous administration, suppressing the amount of the effective ingredient to a low level. The administration of the agents may be carried out by way of intravenous injection or intramuscular injection.

The inhibitory agents of this invention can effectively inhibit HIV superinfection of cells persistently infected with HIV so that the change of such persistently infected cells to cells capable of producing strongly toxic virus by supuerinfection is effectively prevented. The antigen in this invention for producing the anti-idiotype antibody is not only effective for preventing superinfection but also useful as antigen for producing the anti-idiotype antibody constituting the effective ingredient of the inhibitory agents of this invention. In addition, according to the process of this invention using the antigen for producing the anti-idiotype antibody, the anti-idiotype antibody can be easily obtained.

### EXAMPLES OF THE INVENTION

The present invention will now be illustrated more in detail by way of Examples, but it is to be construed that the present invention is not limited by these Exampls.

### EXAMPLE 1

### (1) 〈Preparation of a peptide consisting of an amino acid chain of 66th to 92nd amino acids as counted from N-terminal of human CD4 molecule wherein the Cys of the 84th amino acid is modified with Bzl〉

Glu at the C-terminal of the peptide was converted into its derivative Fmoc-Glu(OBut)-Opfp active ester (1mmol). Using this ester together with 0.2 mmol of DMAP (4-dimethylaminopyridine) as a catalyst, this ester was introduced by way of ester linkage in DMF (dimethylformamide) onto 0.2 mmol of a polyalkoxybenzyl alcohol resin (0.35 meq OH/g, polystyrene 1% divinylbenzene copolymer, Kokusan Chemical Works, Tokyo, Japan).

The protective groups on the side chains of Fmoc-amino acid derivative (Milligen/Biosearch, Division of Millipore Ltd.) used were Asp(OBut), Glu(OBut), Thr (But), Ser(But), Tyr(But), Lys(Boc), His(Boc), Arg(Mtr) and Cys(Bzl). Each condensation reaction at the time of extending the peptide chain was carried out all by using a pfp(pentafluorophenyl) active ester (2.5 eq) and 0.2 mmol HOBT (1-hydroxybenzotriazole) as a catalyst in DMF except for the case of threonine and serine.

Removal of each Fmoc group was performed by using a mixture of 20% by weight of piperidine and dimethylformamide. Each coupling reaction was carried out while monitoring the reaction with ninhydrin. After completion of the whole coupling reaction, Fmoc group remaining at the N-terminal was removed by using a mixture of 20% by weight of piperidine and dimethylformamide to make the NH₂ group free. In order to remove all of the protecting groups and to liberate the peptide from the resin, the mass was treated for 3 hours at room temperature with TFA-thioanisole in the presence of m-cresol. The mixture was filtered with a glass filter to remove the resin and the filtrate was concentrated at room temperature. Ether was then added to obtain a powdery peptide. The resultant powdery peptide was collected and dissolved in an ammonium formate buffer, and the solution was treated with SEPHADEX G-25 (Farmacia Fine Chemicals Co.), washed out with 0.5N acetic acid. After desalting followed by purification, the crude product was further purified by way of high pressure liquid chromatography (HPLC).

Fractionation by HPLC is carried out by setting a concentration gradient of 10 to 60% acetonitrile in 0.1% by weight of TFA and allowing NUCLEOSIL 100 5c18 (Nakarai Tesque, Kyoto, Japan) to flow at a rate of 1 ml/min through a column (4.0 × 150 mm) while monitoring at 210 and 260 nm. A peptide consisting of an amino acid chain of 66th to 92nd amino acids as counted from the N-terminal of human CD4 molecule was eluated at a retention time of 16.8 minutes. A part of the fraction obtained was subjected to an amino acid analysis by the aid of an Amino Acid Analyzer Model No. 835S (Hitachi Mfg. Works, Tokyo, Japan) whereby it was detected that the product was CD4 (66-92, Cys-Bzl) peptide aimed at.

### (2) 〈Preparation of a peptide consisting of an amino acid chain of 68th to 130th amino acids as counted from the N-terminal of human CD4 molecule wherein the Cys of the 84th amino acid is modified with Acm〉

Cys at the C-terminal of the peptide was introduced onto the resin in accordance with the method in the foregoing paragraph (1) and a peptide consisting of an amino acid chain of 68th to 130th amino acids as counted from the N-terminal of human CD4 molecule wherein the Cys of the 84th amino acid was modified with Acm was prepared in the same manner as in (1) using the Fmoc method.

### (3) 〈Preparation of a control peptide consisting of an amino acid chain of 45th to 60th amino acid as counted from the N-terminal of human CD4 molecule〉

Ser at the C-terminal of the peptide was introduced onto the resin in accordance with the method in the foregoing paragraph (1) and a peptide consisting of an amino acid chain of 45th to 60th amino acids as counted from the N-terminal of human CD4 molecule was prepared as a control negative peptide for comparison in the same manner as in (1)

Thr-Lys-Gly-Pro-Ser-Lys-Leu-Asu-Asp-Arg-Ala-Asp-Ser-

Arg-Arg-Ser

### EXAMPLE 2

### 〈Preparation of a monoclonal antibody capable of reacting with a peptide consisting of an amino acid chain of 68th to 130th amino acids or 66th to 92nd amino acids as counted from the N-terminal of human CD4 molecule〉

### (1) Preparation of Immunized mouse spleen cell

A BALB/c female mouse with five weeks of age supplied from Charles Riber Japan, Inc. was immunized by intraperitoneal injection of 500 µg of the human CD4 peptide (68-130) simultaneously with complete Freund's adjuvant. After two weeks, 200 µg of the same peptide dissolved in PBS was administered intraperitoneally for additional immunization. A similar immunization process was repeated three times and, 3 days after the third immunization, the spleen was taken out, disintegrated in RPMI-1640 medium, suspended and washed to produce spleen cells for fusion.

### (2) Preparation of mouse myeloma cells

Mouse myeloma P3-X63-Ag8-U1 was cultivated in a DMEM medium containing 10% of FCS and the cells in the logarithmic growth phase were collected as the parent strain for fusion.

### (3) Cell fusion

The spleen cells (2 × 10⁸) and the mouse myeloma cells (4 × 10⁷) obtained in the above (1) and (2) were mixed and centrifuged into a pellet. After discarding the supernatant, the pellet was disintegrated thoroughly and admixed gradually with 1 ml of 50% polyethylene glycol 1500 (Boehringer Mannheim Inc.) over 1 to 2 minutes to effect cell fusion. Next, 10 ml of the DMEM medium was added dropwise under agitation over about 10 minutes to complete the cell fusion. To the mixture was added 1 ml of FCS to terminate the fusion reaction.

After removing the supernatant polyethylene glycol solution by centrifugal separation, the remaining pellet was wahsed with the DMEM medium containing 10% FCS and the supernatant was removed by centrifugal separation. The pellet was suspended in a HAT culture medium (DMEM culture medium containing 100µM of hypoxanthine, 0.4µM of aminopterin, 16µM of thymidine, 0.03% glutamine and 15% FCS) and poured onto a 96-well microtiter plate at a rate of 0.2 ml per well so that 5 × 10⁵ cells were accommodated in each well. Cultivation was carried out in a carbon dioxide cultivator at 37°C and the HAT culture medium was renewed every other day at a rate of 0.1 ml per well. Cultivation and growth were continued for 7 to 10 days to produce fused cell (hybridoma) colonies.

### [Enzyme Immunoassay]

A solution of human CD4 peptide (1µg/ml) dissolved in a phosphate buffered saline (PBS) was poured onto a 96-well microtiter plate for ELISA (Sumitomo Bakelite Co., Ltd., Tokyo, Japan) in an amount of 50 µl/well and allowed to stand at room temperature for 1 to 4 hours thereby permitting the peptide to be absorbed onto the bottom surface of each well. After washing each well three times with a phosphate buffer solution (PBS, pH 7.4) containing 0.15 M of sodium chloride, PBS containing 3% of casein sodium was charged into each well and allowed to stand for 1 hour at room temperature for coating the plate wells fully with casein. After discarding the liquid in each well and washing with PBS, a supernatant liquid of the hybridoma culture was added in an amount of 50 µl/well and allowed to stand at room temperature for 1 to 4 hours. After washing each well 5 times with PBS containing 0.05% TWEEN 20, a solution of an anti-mouse IgG antibody bound with peroxidase (Nippon Bio-Rad Lab., Japan), diluted to a concentration of 1/4000 with PBS was poured in an amount of 50 µl/well and allowed to stand at room temperature for 1 to 2 hours. After washing 5 times with 0.05% TWEEN 20-PBS and twice with PBS, a coloring liquid prepared by mixing 50 mM of sodium acetate buffer (pH 5.0) containing 10 mM of o-phenylenediamine and 0.025% BSA with 0.1% aqueous solution of hydrogen peroxide in a ratio of 3:1 was added to each well in an amount of 50 µl/well and allowed to stand for 15 to 30 minutes at room temperature to effect reaction. 1N sulfuric acid containing 0.1% sodium sulfite (Na₂SO₃) was added to each well in an amount of 150 µl/well to cease the reaction, and light absorbance of the hybridoma was measured at a wavelength of 492 nm. On screening the resultant hybridoma, strains in the supernatant liquid showing a high value in measurements was selected.

### (4) Screeing and Cloning of the Hybridomas

The supernatant liquid of each well was taken up and used for the above mentioned enzyme immunoassay wherein human CD4 peptides (68-130) and (66-92) were used as antigens. The hybridomas in the wells showing production of antibody to the above antigen were subjected to cloning according to the limiting dilution method whereby clone strains (CP-9, CP-1) were obtained which produced anti-human CD4 peptide (68-130) antibody and anti-human CD4 peptide (66-92) antibody, respectively.

### (5) Purification of Monoclonal Antibody

Each 50 ml of the hybridoma strains obtained in the above (4) was cultivated in a non-serum culture medium ASF-103 (Ajinomoto Co., Ltd.). The cultured products wherein the cell concentration became 1 × 10⁶/ml were subjected to centrifugal separation, the supernatant liquids obtained were then subjected to chromatographic treatment using PROTEIN A-AGAROSE (Nippon Bio-Rad Lab., Japan) or LENTILECTIN-SEPHAROSE (Pharmacia Fine Chemicals Co.). In the former case, a citric acid buffer solution of pH 4.0 was used as an eluting liquid, while in the later case PBS containing 10% methyl-α-D-mannopyranoside was used as an eluting liquid. The resultant eluates were dialyzed whereby the purified monclonal antibodies aimed at were obtained.

### EXAMPLE 3

### 〈Preparation of Anti-idiotype Antibody produced from Monoclonal Antibody as Immunugen capable of reacting with a peptide consisting of an amino acid chain of 68th to 130th amino acids as counted from the N-terminal of human CD4 molecule〉

### (1) Immunization

Each BALB/c female mouse of 5 weeks old was immunized by way of intraperitoneal administration with the purified IgG sample of the monoclonal antibody #63 (produced by hybridoma CP-9 strain) capable of reacting with human CD4 peptide (68-130) or the monoclonal antibody #35 (produced by hybridoma CP-1 strain) capable of reacting with human CD4 peptide (66-92), each peptide obtained in Example 2 together with an aluminum adjuvant. Each mouse was immunized 4 times by every 2 weeks, each time with 100 µg of IgG and, after 2 weeks, subjected to additional immunization with 200 µg of IgG. The spleen was extracted 3 days after the additional immunization.

### (2) Preparation of Hybridoma

In the same manner as illustrated in Example 2, immunized mouse spleen cells and myeroma cells were prepared and cell fusion was carried out using these cells.

### (3) Screening and Cloning of Hybridomas

Screening of hybridoma was carried out by way of enzyme immunoassay just as in Example 2. In 1 ml of 0.2 M sodium acetate buffer solution (pH 4.5) were dissolved 10 mg of IgG sample of #63 or #35 (monoclonal antibodies subjected to immunization) and 100 µg of pepsin (Sigma Chemical Co., St. Louis, U.S.A.). The solution was kept at 37°C for 20 hours to effect reaction, thereby performing limitative decomposition. After adjusting pH to 8, the reaction mixture was subjected to gel filtration through a SEPHADEX G-150 (Pharmacia Fine Chemicals Co.) column to obtain a limitative decomposition product F (ab')₂ of IgG in a fraction of molecular weight of about 100,000. The product F (ab')₂ of #35 or # 63 dissolved in PBS (2 µg/ml) was adsorbed on ELISA plates (Costar Corp., Cambridge, Ma., U.S.A.) at 37°C for 80 minutes and used as a solid phase antigen. After coating with casein, the supernatant liquid of each hybridoma culture was added onto the plate in an amount of 50 µl/well and reacted at 37°C for 60 minutes. After washing, a peroxidase-bound anti-mouse IgG (Fc) antibody (Organon Teknika Corp., West Chester, Pa. U.S.A.) diluted to 1/4000 with PBS was added in an amount of 50 µl/well and reacted at 37°C for 60 minutes. After coloration was carried out as in Example 2, a hybridoma strain was selected which produced a supernatant liquid showing a higher value of measurement was selected. Each hybridoma was subjected to cloning according to the limiting dilution method and then to screening as described above whereby each clone strain capable of producing each anti-idiotype antibody (ID55 prepared from #63 as an immunogen and ID 62 prepared from #35 as an immunogen) was obtained. Each anti-idiotype antibody produced from each strain was purified in the same manner as described in Example 2.

### PREPARATION EXAMPLE

### Preparation of L-2 cells

At the outset, LAV-1 virus (F. Barre Sinoussi *et al*., Science, 220, 868-871, 1983) was prepared as follows: MOLT-4 cells persistently infected with LAV-1 were suspended in an RMPI-1640 medium containing 10% fresh FCS so that the medium involved 5 × 10⁵ cells/ml. The cells were then cultivated in a CO₂ incubator at 37°C for 3 days and LAV-1 virus particles contained in the medium were recoverd as a supernatant liquid obtained by centrifugal separation (100 g × 5 min.). This liquid was stored as an LAV-1 stock solution at -85°C. The titer of this LAV-1 virus stock solution was then determined by using MT-4 cells. More precisely, the virus liquid was diluted with an RPMI-1640 medium to 1/1 × 10 to 1 × 10⁸, and the diluted medium (100 µl) was poured as a layer for mixing with a MT-4 cell liquid supended in an RPMI-1964 medium containing 20% FCS to 1 × 10⁶ cells/ml and poured into a 96-well microplate in an amount of 100 µl/well. After incubation for 4 days, the liquid was coated on a slide glass and fixed with cooled acetone (-20°C) for 5 minutes for detecting infection of MT-4 cells with LAV-1 by way of the fluorescence antibody method. This sample was reacted with a mouse monoclonal antibody liquid (V17) (Ikuta *et al*.,Virol. 170, 408-417, 1989) as a primary antibody capable of specifically detecting P18 protein of HIV at room temperature for 15 minutes, washed with PBS and reacted with an anti-mouse IgG antibody (Organon Teknika Corp., West Chester, Pa., U.S.A.) bound with fluoresein isothiocyanate (FITC) as a secondary antibody diluted with PBS to 1/40 at room temperature for 15 minutes and washed with PBS. The titer was determined by the final value of the LAV-1 virus liquid being diluted at which a fluorescence specific to FITC was detected by a fluorescence microscope. More specifically, the 10 times value of the final dilution value was decided as an infectious titer per ml,i.e. TCID₅₀/ml (Tissue Culture Infectious Dose 50).

MT-4 cells in an RPMI-1640 culture medium containing 10% FCS in the logarithmic growth phase were collected by way of centrifugal separation and suspended in an RPMI-1640 culture medium containing 20% fresh FCS so that the medium contained 1 × 10⁶ cells/ml. To 1 ml of the MT-4 cell liquid was added 1 ml of the LAV-1 virus liquid diluted with an RPMI-1640 culture medium so that the titer TCID₅₀/ml was equal to 10⁶, and the mixture was incubated at 37°C for 1 hour. The MT-4 cells were collected by centrifugal separation and washed twice with RPMI-1640 medium. In this case, one MT-4 cell was infected with one LAV-1 virus so that M.I.O. (multiplicity of infection) value at the time of infection with LAV-1 was 1. As a first method, the cells were immediately subjected in RPMI-1640 medium containing 20% FCS to cloning by the limiting dilution method, poured into a 96-well microplate in an amount of 1 cell/0.2 ml/well and cultivated for 2 weeks in a CO₂ incubator.

As a second method, the washed cells were suspended in an RPMI-1640 culture medium containing 10% FCS so that the suspension contained 1 × 10⁶ cells/ml and cultivated in a CO₂ incubator. During 3 to 7 days from the initiation of cultivation, strong cytopathic effect was observed which was caused by infection with LAV-1, and a great number of the cells were killed. After that, cells persistently infected with LAV-1 initiated to propagate logarithmically similarly to non-infected cells. The culture medium was replaced on alternate day with a fresh culture medium so that the medium might always contain 1 × 10⁶ cells/ml. The cells were then subjected to cloning in an RPMI-1640 culture medium containing 20% FCS according to the limiting dilution method, and the medium was poured onto a 96-well microplate in an amount of 1 cell/0.2 ml/well and cultivated in a CO₂ incubator for 2 weeks.

As a result of mass-culturing clone cells of MT-4 cells persistently infected with LAV-1, which were multiplicated in the microplate, and making search for HIV phenotypes in terms of the formation of core (gag) and envelope (env) proteins, infectivity and reverse transcription enzyme (RT) activity, variation was found in various phenotypes. For example, there were found gag protein not cleaved with precursor protein p53 being kept, env protein not cleaved with precursor protein gp160 being kept, cells showing no RT activity, and a combination thereof. As a result, 85% of the whole showed no infectivity. Among these, cells featured by the phenotype negative in cleavage of precursor protein p53 of the gag protein and negative in RT activity (referred to hereinafter as L-2 cells) were found. In the fluorescence method, the L-2 cells reacted with ripened gag protein p18 but did not react with anti-P18 mouse monoclonal antibody (V17) incapable of reacting with precursor gag protein p53. Further, L-2 cells were negative in RT activity as a result of the test that using the reaction liquid containing [³²P]dTTP (NEN, E. I. DuPont, Wilmington, Del., U.S.A.) oligo dT, poly rA (Pharmacia-LKB), the produced [³²P) poly T DNA was fixed on DEAE paper (trade name: DE81, Wattman Corp.) and radioactivity on the DEAE paper was detected by way of autoradiography using an X-ray film (Kodak Co., U.S.A.).

As host cells for investigating occurrence of superinfection and inhibitory effect thereon, those just like the above L-2 cells are preferable which are of sharp difference in phenotype and are easy and simple for measurements. However, the host cells are not limited to L-2 cells. Occurrence of superinfection can be judged as to whether or not such variation is restored to normal state and therefore occurrence of superinfection will be determined if the fluorescence antibody method using V17 antibody becomes positive or the RT activity becomes positive.

### TEST EXAMPLE

### (1) Superinfection of L-2 cells with HTLV-IIIB

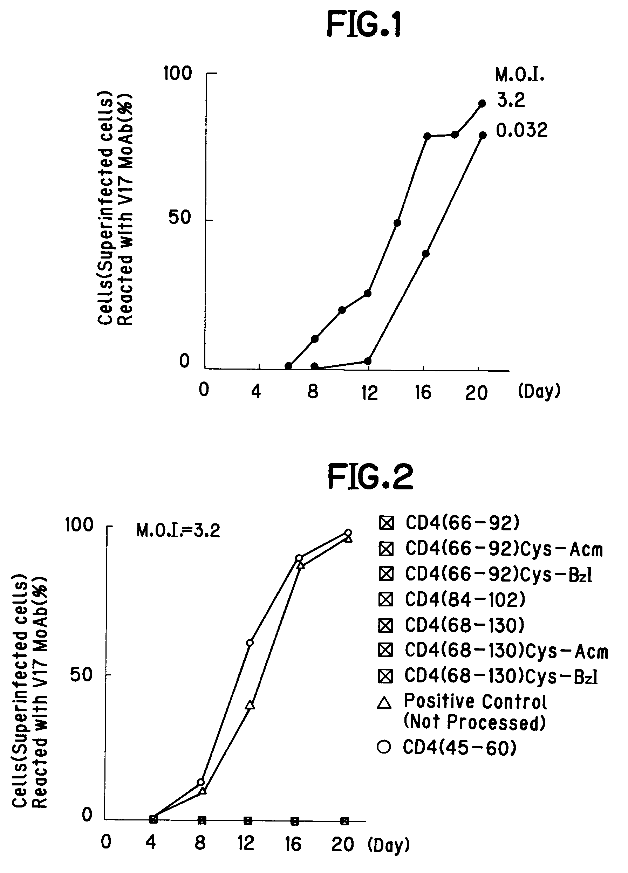

L-2 cells were supended in an RPMI-1640 culture medium containing 20% FCS so that the medium contained 1 × 10⁶ cells/ml, and added to a 96-well microplate in an amount of 50 µl/ml. The medium was then overlaid with a HTLV-IIIB virus (Popovic *et al*., Science, 224, 497-500, 1984) liquid corresponding to M.O.I. (multiplicity of infection) = 3.2 or 0.032 in an amount of 50 µl/well, so that the cells were infected. The supernatant culture liquid and culture cells were extracted every 4 days and each extracted sample was checked for RT activity and the reaction with p18 antigen to make them as indices of superinfection. The presence or absence of the formation of p18 antigen is shown in Fig. 1.

### (2) Inhibition of superinfection by various CD4 reagents

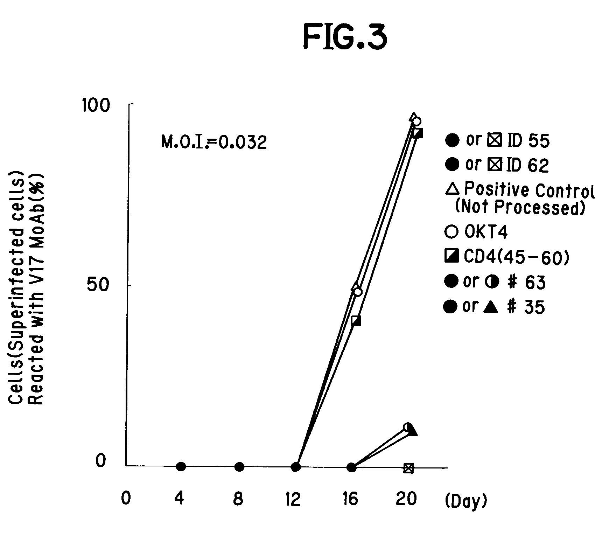

Each of the CD4 peptides [45-60, 66-92, 66-92(Cys-Bzl), 66-92(Cys-Acm), 84-102, 68-130, 68-130(Cys-Bzl) and 68-130 (Cys-Acm)] prepared in Example 1 was dissolved in an RPMI-1640 culture medium so that the final concentration was 1 mg/ml, and each solution was mixed with an HTLV-IIIB virus liquid corresponding to M.O.I.= 3.2. The mixture was reacted together at room temperature for 30 minutes, and L-2 cells were inoculated with the reacted mixture as in the above (1). Further, mouse anti-CD4 monoclonal antibody [OKT4 (Ortho Diagnostic Systems, Inc.)], mouse monoclonal antibody [#35 (produced by Hybridoma CP-1 strain) and #63 (produced by Hybridoma CP-9 strain)] against CD4 (68-130) and CD4 (66-92) prepared in Example 2, and anti-idiotype antibody ID55 (produced from #63 as an immunogen) and antiidiotype antibody ID62 (produced from #35 as an immunogen) were, respectively, diluted with an RPMI-1640 culture medium containing 20% PBS so that the final concentration was 5 µg/ml (OKT4) or 200 µg/ml (#35, #63, ID55 or ID62), and treated, as in the above (1), with L-2 cells at room temperture for 30 minutes. Each mixture thus treated was inoculated with the virus liquid corresponding to M.O.I. of 0.032 (OKT4, #35 and #63) or was mixed with the above virus liquid, treated at room temperature for 30 minutes and then inoculated into L-2 cells (ID55 and ID62). After one day, these cells were washed with each fresh culture medium and occurrence of superinfection was checked every 4 days as in the above (1). Fig. 2 show the result in the case of M.O.I. being 3.2, while Fig. 3 shows the result in the case of M.O.I. being 0.032.

### (3) Results

### (a) Superinfection of L-2 cells with HTLV-IIIB

L-2 cells were infected with HTLV-IIIB corresponding to M.O.I. of 3.2 or 0.032. Continuing culturing of the cells, reactivity against V17 anti-p18 monoclonal antibody became noticeable from about 8th day of the culturing (Fig. 1). According to the measurement of RT activity by way of autoradiography, it was observed that RT activity was detected in the supernatant liquid of the culture medium almost simultaneously with the reactivity against V17 anti-p18 monoclonal antibody, thus showing occurrence of superinfection. On superinfection, there was no cytopathic activity as observed at the time of infection of MT-4 cells with HTLV-IIIB, but production of infectious wild-type HIV particles was observed. The velocity of superinfection was, as shown in Fig. 1, such that about 16 days were required until 80% positive reaction was exhibited even in the case of M.O.I. being 3.2. In case MT-4 cells were inoculated with the same virus liquid, the reaction become positive more then 80% within 4 days. It has been noted therefore that spreading of superinfection needed a long period time.

### (b) Effectiveness of CD4 peptide against superinfection

Superinfection was completely inhibited when CD-4 peptides (68-130, Cys-Acm) or (66-92, Cys-Bzl) was added at the final concentration of 1mg/ml (Fig. 2). However, such inhibitory effect was not at all observed in case of CD-4 peptide (45-60) at the same concentration. Namely, the inhibitory effect on superinfection was an extremely specific function in CD4 peptide in the region of (66-92), (66-92, Cys-Acm), (66-92, Cys-Bzl), (84-102), (68-130), (68-130, Cys-Acm) and (68-130,Cys-Bzl). Fig. 2 shows the result obtained in case of using V-17 antibody according to the fluorescence antibody method, but a similar result is obtained in RT activity as well.

### (c) Effectiveness against superinfection of monoclonal antibody and anti-idiotype antibody against CD4 peptide

Among the anti-CD4 monoclonal antibodies, OKT4 devoid of inhibitory effect against infection with HIV was not observed to possess inhibitory effect against superinfection (Fig.3). However, the monoclonal antibody #35 or #63 serves to delay and inhibit superinfection at the final concentration of 200 µg/ml (Fig. 3). It is also noted that anti-idiotype antibody ID55 or ID62 completely inhibits superinfection at the final concentration of 200 µg/ml (Fig. 3).

## Claims

1. An inhibitory agent against HIV superinfection comprising an effective amount of a peptide consisting of an amino acid chain of 66th to 92nd amino acids as counted from N-terminal of human CD4 molecule whereby after cells are initially infected with HIV, reinfection with second-round HIV is prevented.

2. An inhibitory agent against HIV superinfection comprising an effective amount of a peptide consisting of an amino acid chain of 68th to 130th amino acids as counted from N-terminal of human CD4 molecule whereby after cells are initially infected with HIV, reinfection with second-round HIV is prevented.

3. An inhibitory agent against HIV superinfection comprising an effective amount of a peptide consisting of an amino acid chain of 84th to 102nd amino acids as counted from N-terminal of human CD4 molecule whereby after cells are initially infected with HIV, reinfection with second-round HIV is prevented.

4. An inhibitory agent against HIV superinfection comprising an effective amount of a peptide consisting of an amino acid chain of 66th to 92nd amino acids as counted from N-terminal of human CD4 molecule wherein a sulfydryl group of cysteine reside of the 84th amino acid is modified with an acetamidomethyl group, whereby after cells are initially infected with HIV, reinfection with second-round HIV is prevented.

5. An inhibitory agent against HIV superinfection comprising an effective amount of a peptide consisting of an amino acid chain of 66th to 92nd amino acids as counted from N-terminal of human CD4 molecule wherein a sulfydryl group of cysteine reside of the 84th amino acid is modified with a benzyl group, whereby after cells are infected with HIV, reinfection with second-round HIV is prevented.

6. An inhibitory agent against HIV superinfection comprising an effective amount of a peptide consisting of an amino acid chain of 68th to 130th amino acids as counted from N-terminal of human CD4 molecule wherein a sulfydryl group of cysteine residue of the 84th amino acid is modified with an acetamidomethyl group, whereby after cells are initially infected with HIV, reinfection with second-round HIV is prevented.

7. An inhibitory agent against HIV superinfection comprising an effective amount of a peptide consisting of an amino acid chain of 68th to 130th amino acids wherein a sulfydryl group of cysteine residue of the 84th amino acid is modified with a benzyl group, whereby after cells are initially infected with HIV, reinfection with second-round HIV is prevented.

8. An inhibitory agent against HIV superinfection comprising an effective amount of a monoclonal antibody capable of reacting with a peptide consisting of an amino acid chain of 66th to 92nd amino acids as counted from N-terminal of human CD4 molecule, whereby after cells are initially infected with HIV, reinfection of second-round HIV is prevented.

9. An inhibitory agent against HIV superinfection comprising an effective amount of a monoclonal antibody capable of reacting with a peptide consisting of an amino acid chain of 68th to 130th amino acids as counted from N-terminal of human CD4 molecule, whereby after cells are initially infected with HIV, reinfection of second-round HIV is prevented.

10. An antigen for producing an anti-idiotype antibody comprising an antibody capable of reacting with a peptide consisting of an amino acid chain of 66th to 92nd amino acids as counted from N-terminal of human CD4 molecule, said anti-ideotype entibody functioning so that after cells are initially infected with HIV, reinfection with second-round HIV is prevented.

11. An inhibitory agent against HIV superinfection comprising an effective amount of an anti-idiotype antibody produced from the antigen for producing anti-idiotype antibody defined in claim 10, whereby after cells are initially infected with HIV, reinfection with second-round HIV is prevented.

12. A process for producing an anti-ideotype antibody which comprises producing the anti-ideotype antibody from an antibody as an antigen reactable with a peptide consisting of an amino acid chain of 66th to 92nd amino acids as counted from N-terminal of human CD4 molecule, said anti-ideotype antibody functioning so that after cells are initially infected with HIV, reinfection of second-round HIV is prevented.

13. An antigen for producing an anti-idiotype antibody comprising of an antibody capable of reacting with a peptide consisting of an amino acid chain of 68th to 130th amino acids as counted from N-terminal of human CD4 molecule, said anti-ideotype antibody functioning so that after cells are infected with HIV, reinfection with second-round HIV is prevented.

14. An inhibitory agent against HIV superinfection comprising an effective amount of an anti-idiotype antibody produced from the antigen for producing an anti-idiotype antibody defined in claim 13, whereby after cells are initially infected with HIV, reinfection with second-round HIV is prevented.

15. A process for producing an anti-idiotype antibody comprising producing the anti-ideotype antibody from an antibody as an antigen reactable with a peptide consisting of an amino acid chain of 68th to 130th amino acids as counted from N-terminal of human CD4 molecule, said anti-ideotype antibody functioning so that after cells are initially infected with HIV, reinfection of second-round HIV is prevented.
